# EUROPEAN PATENT APPLICATION

(11) **EP 4 353 284 A1**
(43) Date of publication of application: **17.04.2024**
(21) Application number: 22819517.8
(22) Date of filing: 07.06.2022
(51) Int. Cl.: A61M 11/00, A61M 15/00

(54) **MICRO ATOMIZATION DEVICE**

(30) Priority: 09.06.2021 CN 202110642191
(71) Applicant: Suzhou Skywell Healthcare Information Co., Ltd., Suzhou, Jiangsu 215123 (CN)
(72) Inventor: GU, Yu, Suzhou, Jiangsu 215123 (CN)
(74) Representative: Simmons & Simmons LLP (Munich)
(86) International application number: PCT/CN2022/097364
(87) International publication number: WO 2022/257919

(57) **Abstract**

An atomization device (100, 200, 300), comprising: a liquid conveying unit (102, 302), the liquid conveying unit (102, 302) comprising a liquid storage container (122, 322) having a liquid outlet (121), the liquid conveying unit (102, 302) being configured to output a liquid to be atomized from the liquid outlet (121) of the liquid storage container (122, 322) at a flow rate; an atomization unit (101, 301), the atomization unit (101, 301) being configured to receive the liquid to be atomized from the liquid outlet (121) of the liquid storage container (122, 322) and atomize the liquid to be atomized into a spray, and the atomization unit (101, 301) having a predetermined atomization rate; and a control unit, the control unit being in communication connection with the liquid conveying unit (102, 302), and being configured to control the flow speed of the liquid to be atomized output by the liquid conveying unit (102, 302) according to the predetermined atomization rate of the atomization unit (101, 301).

## Description

### TECHNICAL FIELD

The present application relates to the technical field of atomization, and more particularly, to a micro-atomizing device.

### BACKGROUND

There is a need for automizing a trace of liquid in many fields in current production and scientific research, such as inhalation delivery of a trace of drugs and atomization spraying on the surfaces of micro components. However, currently common atomizers, such as ultrasonic atomizers, screen type atomizers, and air compression atomizers, are often difficult to meet the atomization requirements of such trace of liquid. For reasons, on one hand, some existing atomizers usually adopt a continuous atomization working mode of several minutes or longer, and the amount of atomized liquid is relatively large, so that when a trace of liquid needs to be processed for atomization, the control mode of these existing atomizers often cannot realize accurate control of the atomization amount. For example, some atomizing devices often form an instantaneous voltage pulse when they are started, which will affect the stability of the initial atomization. and this pulse change will significantly influence the atomization amount in a micro-atomization process of several seconds or tens of seconds. On the other hand, due to the fact that the existing atomizers are often having a minimum working liquid level mechanism, residual liquid is inevitably left in the working process of these atomizers. However, reagents or liquids used in the field of micro-atomization tend to have the characteristics of high value and high concentration, and the existence of residual liquid will cause unnecessary waste.

Therefore, there is a need for further improvement of a micro-atomizing device.

### SUMMARY

The objective of the present application is to provide an atomizing device, which can solve the problem of atomizing a trace of liquid.

According to an aspect of the present application, an atomizing device is provided. The atomizing device comprising: a liquid conveying unit, the liquid conveying unit comprising a liquid storage container having a liquid outlet, and the liquid conveying unit being configured to output a liquid to be atomized from the liquid outlet of the liquid storage container at a flow rate; an atomizing unit, wherein the automizing unit is configured to receive the liquid to be atomized from the liquid outlet of the liquid storage container and atomize the liquid to be atomized into mist, wherein the atomizing unit has a predetermined atomization rate; and a control unit, the control unit being in communication with the liquid conveying unit, and the control unit being configured to control a flow rate at which the liquid conveying unit outputs the liquid to be atomized according to a predetermined atomization rate of the atomizing unit.

In some embodiments, there is a gap between the liquid outlet of the liquid storage container and the atomizing unit, the gap allows the liquid to be atomized to form a droplet of a predetermined size at the liquid outlet, and the droplet contacts the atomizing unit and is transferred to the atomizing unit.

In some embodiments, the gap between the atomizing unit and the liquid outlet is insufficient to enable the liquid to be atomized to form a droplet falling due to gravity.

In some embodiments, the control unit is configured to adjust a size of the gap between the atomizing unit and the liquid outlet.

In some embodiments, the control unit is configured to change the size of the droplet that can be formed at the liquid outlet by adjusting the size of the gap between the atomizing unit and the liquid outlet.

In some embodiments, the atomizing unit comprises a piezoelectric ceramic microporous atomization piece, the piezoelectric ceramic microporous atomization piece has a liquid storage area for receiving a liquid to be atomized from the liquid outlet of the liquid storage container, and the flow rate of the liquid to be atomized outputted by the liquid conveying unit is set such that the maximum height of the liquid level in the liquid storage area is 1 mm to 4 mm when the piezoelectric ceramic microporous atomization piece is in a continuous working state.

In some embodiments, the liquid conveying unit comprises a plunger and a plunger rod, wherein the plunger is slidably engaged with the inner wall of the liquid storage container, and the plunger rod is connected to the plunger and is configured to drive the plunger to move in the liquid storage container, so that the liquid to be atomized can flow out of the liquid outlet.

In some embodiments, the atomizing device further comprises: a driving unit configured to drive the plunger rod through a transmission member, wherein the transmission member comprises a lead screw and a lead screw nut, the driving unit is connected to the lead screw to drive the lead screw to rotate, and the plunger rod is connected to the lead screw nut and moves along the axial direction of the lead screw along with the lead screw nut.

In some embodiments, the atomizing device further includes a mist temporary storage container having a mist inlet and a mist suction port, and the mist inlet is configured to be detachably engaged with the atomizing unit to receive and temporarily store the mist atomized by the atomizing unit.

In some embodiments, the atomizing device comprises a concentration mark provided in the mist temporary storage container, and the mist temporary storage container comprises an observation part that can be observed from the outside, and wherein the concentration mark is configured to be visually observed at the observation part when the mist concentration in the mist temporary storage container is lower than a predetermined concentration value, and cannot be visually observed at the observation part when the mist concentration in the mist temporary storage container is higher than the predetermined concentration value.

In some embodiments, the concentration mark is a mark disposed on an inner wall of the mist temporary storage container, and the observation portion is formed by a light-transmissive material.

In some embodiments, the liquid storage container of the liquid conveying unit comprises a liquid level sensor, the control unit is in communication with the liquid level sensor and is configured to generate a warning signal when the liquid level sensor indicates that the liquid in the liquid storage container is lower than a specified liquid level.

In one aspect of the present application, a method for atomizing an apparatus according to any one of the above embodiments is provided, the method comprising the following steps performed by the control unit: determining an atomization rate of the atomizing unit; determining the flow rate of the liquid to be atomized from the liquid outlet of the liquid storage container on the basis of the atomization rate of the atomizing unit; and controlling the liquid conveying unit to output the liquid to be atomized from the liquid outlet of the liquid storage container on the basis of the determined flow rate of the liquid to be atomized.

In some embodiments, a gap is formed between the liquid outlet of the liquid storage container and the atomizing unit, the gap allows the liquid to be atomized to form a droplet of a predetermined size at the liquid outlet, and the droplet contact the atomizing unit and is transferred to the atomizing unit, and the method comprises the following steps performed by the control unit: adjusting the size of the gap between the atomizing unit and the liquid outlet to change the size of the droplet that can be formed at the liquid outlet.

In some embodiments, the atomizing device further includes a mist temporary storage container having a mist inlet and a mist suction port, and the mist inlet is configured to be detachably engaged with the atomizing unit to receive and temporarily store the mist atomized by the atomizing unit; and a concentration mark provided in the mist temporary storage container, and the mist temporary storage container comprises an observation part that can be observed from the outside, the concentration mark being configured to be visually observed at the observation part when the mist concentration in the mist temporary storage container is lower than a predetermined concentration value, and not to be visually observed at the observation part when the mist concentration in the mist temporary storage container is higher than the predetermined concentration value, wherein the method further comprises: and engaging the mist inlet of the mist temporary storage container with the atomizing unit to receive and temporarily store the mist atomized by the atomizing unit; observing the concentration mark through the observation part, and removing the mist temporary storage container from the atomizing unit when the concentration mark cannot be visually observed from the observation part; and sucking the mist temporarily stored in the mist temporary storage container from the mist suction port of the mist temporary storage container.

The foregoing is an overview of the present application, which may be simplified, summarized, and omitted, and thus those skilled in the art will recognize that this portion is illustrative only and is not intended to limit the scope of the present application in any manner. This Summary is not intended to identify key features or essential features of the claimed subject matter, nor is it intended to be used as an auxiliary means to determine the scope of the claimed subject matter.

### BRIEF DESCRIPTION OF THE DRAWINGS

The foregoing and other features of the present application will become more fully apparent from the following description and the appended claims, and in conjunction with the accompanying drawings. It can be understood that these drawings depict only several embodiments of the present application, and thus should not be considered as limiting the scope of the present application. By adopting the accompanying drawings, the content of the present application will be described more clearly and in detail.
FIG. 1 is a schematic diagram of an atomizing device 100 according to an embodiment of the present application.
FIG. 2 is a system schematic block diagram of an atomizing device 200 according to another embodiment of the present application.
FIG. 3 is a schematic diagram of an atomizing device 300 according to yet another embodiment of the present application.
FIG. 4 illustrates a flowchart of a method 400 of using an atomizing device according to an embodiment of the present application.

### DETAILED DESCRIPTION

The following detailed description refers to the accompanying drawings that form a part of the description. In the drawings, similar symbols generally represent similar components unless the context dictates otherwise. The illustrative embodiments described in the detailed description, drawings, and claims are not intended to be limiting. Other embodiments may be employed and other changes may be made without departing from the spirit or scope of the subject matter of the present disclosure. It may be understood that various configurations, substitutions, combinations, and designs of various configurations may be made in various aspects of the present disclosure generally described in this application and illustrated in the accompanying drawings, all of which explicitly constitute a part of the present disclosure.

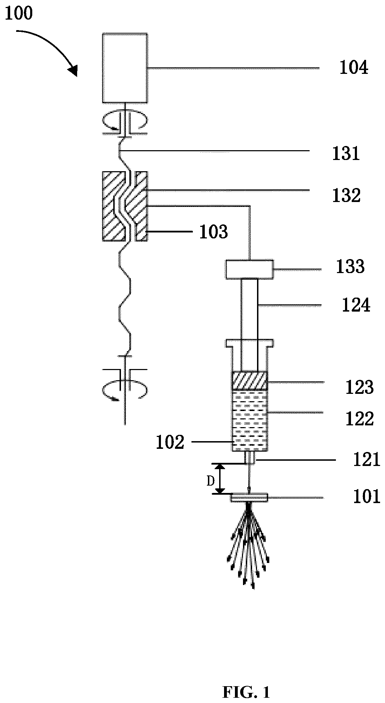

FIG. 1 is a schematic diagram of an atomizing device 100 according to an embodiment of the present application. In some embodiments, the atomizing device may be used to atomize a trace of liquid, such as 1 µl to 5 ml.

As shown in FIG. 1, the atomizing device 100 includes an atomizing unit 101 and a liquid conveying unit 102, wherein the liquid conveying unit 102 is configured to deliver a liquid to be atomized to the atomizing unit 101, and the atomizing unit 101 receives the liquid to be atomized and atomizes the liquid to be atomized into a liquid mist. The liquid conveying unit 102 includes a liquid storage container 122 for storing a liquid to be atomized, the liquid storage container 122 is having a liquid outlet 121, and the liquid conveying unit 102 is configured to output the liquid to be atomized in the liquid storage container 122 from the liquid outlet 121 at a certain flow rate. It should be noted that, the " certain flow rate " described herein may include continuously outputting the liquid to be atomized at a certain rate or outputting a certain amount of the liquid to be atomized at a certain frequency intermittently, so as to output the amount of the liquid to be atomized in a unit time. In addition, the liquid conveying unit 102 may take any available liquid delivery form, with regard to its delivery means and related structures will be described in more detail below.

The atomizing unit 101 shown in FIG. 1 may employ any available atomizing structure form, such as a solid-hole ceramic atomizing piece, a microporous ceramic atomizing piece, a jet atomizer, and the like. In some embodiments, the atomizing unit 101 may have a predetermined atomization rate. It can be understood that the atomizing unit 101 may be driven by electricity or other means, and the atomization rate of the atomizing unit 101 may be adjusted with different driving voltage/current/power or other driving forces. Although not shown in FIG. 1, the atomizing device 100 may further include a control unit, the control unit is in communication with the liquid conveying unit 102, and the liquid conveying unit 102 is controlled to output the liquid to be atomized at a flow rate according to the predetermined atomization rate of the atomizing unit 101. In this way, the atomizing device 100 can enable the amount of the liquid to be atomized received by the atomizing unit 101 to be always within a suitable range, and the liquid conveyed by the liquid conveying unit 102 can be atomized by the atomizing unit 101 in time, thereby ensuring the atomization efficiency while avoiding excessive liquid on the atomizing unit 101 to affect the atomization effect.

Taking the atomizing unit 101 employing a piezoelectric ceramic microporous atomizing piece as an example, the piezoelectric ceramic microporous atomizing piece has a liquid storage area (not shown) for receiving the liquid to be atomized from the liquid outlet 121. The control unit can control the liquid conveying unit 102 to output the to-be-atomized liquid from the liquid outlet 121 at a flow rate according to the atomization rate of the piezoelectric ceramic microporous atomizing piece, so that the liquid film thickness of the to-be-atomized liquid in the liquid storage area of the microporous atomizing piece is always in a proper range, for example, the maximum height of the liquid level formed in the liquid storage area is 1 mm to 4 mm, so that the low atomization efficiency caused by too small liquid amount in the liquid storage area is avoided, and the atomization effect and efficiency are also prevented from being influenced by excessive liquid volume in the liquid storage area. The above manner is particularly effective for an application scenario in which a trace of liquid is atomized multiple times or continuously.

It should be noted that the liquid storage area may be any structure configured on the atomizing unit and used for bearing a certain amount of liquid to be atomized. In some embodiments, the liquid storage area may be a cylindrical recess, a spherical recess, a cuboid recess, a truncated cone-shaped recess, or the like. In other embodiments, the liquid storage area may be a spherical crown-shaped recessed structure formed on the inner concave spherical crown surface. In still other embodiments, the liquid storage area may also be a combination of some recessed structures, such as a combination of a cylindrical recess and a spherical recess. In some other embodiments, the liquid storage area may also be a structure or a unit that protrudes most of the area of the atomizing unit.

It should be noted that the control unit may obtain the predetermined atomization rate of the atomizing unit 101 in various manners. In some embodiments, the control unit may obtain the predetermined atomization rate of the atomizing unit 101 input by the operator through the human-computer interaction interface. In some other embodiments, the atomizing unit 101 may have an identifiable mark (such as a radio frequency tag, etc.) and is detachably attached to the atomizing device 100, and the control unit may obtain information of a predetermined atomization rate of the atomizing unit 101 by identifying the corresponding mark. In this way, after the user replaces the corresponding atomizing unit 101 according to different atomization requirements, the control unit can accurately identify the predetermined atomization rate of the replacement atomizing unit 101, and correspondingly adjust the speed of the liquid conveying unit 102 outputting the liquid to be atomized.

Still referring to FIG. 1, there is a gap D between the liquid outlet 121 of the liquid storage container 122 and the atomizing unit 101, and the gap D is configured such that the liquid to be atomized can form a droplet of a predetermined size at the liquid outlet 121, and the droplet of the predetermined size can contact the atomizing unit 101 (for example, the bottom surface, the side wall or the edge of the liquid storage area) and is transferred to the atomizing unit 101 due to the liquid tension. The gap described herein is the closest distance between the liquid outlet 121 and the atomizing unit 101. In some embodiments, the gap D between the atomizing unit 101 and the liquid outlet 121 may be configured to be insufficient to enable the liquid to be atomized to form a droplet falling due to gravity. For example, depending on the shape, aperture, or other settings of the liquid outlet 121, the size of the liquid droplet of the liquid to be atomized formed by the liquid outlet 121 is 10 microliters. Then, in order to avoid free falling due to gravity, the size of the gap D between the atomizing unit 101 and the liquid outlet 121 may be 1.34 mm or less (assuming that the droplet is generally circular and corresponds to a volume of 10 microliters). In this way, the to-be-atomized liquid outputted from the liquid outlet 121 has been in contact with the atomizing unit 101 and is transferred to the atomizing unit 101 before forming a droplet falling due to gravity, and thus the problem of low conveying efficiency caused by conveying the droplet by gravity can be avoided. Meanwhile, the droplet is prevented from splashing out of the effective atomization area of the atomizing unit 101 due to gravity falling. In this way, the liquid conveying efficiency between the liquid conveying unit 102 and the atomizing unit 101 can be effectively improved, especially in an application scenario of micro-liquid conveying. In some embodiments, the gap D between the liquid outlet 121 and the atomizing unit 101 is set to not greater than 2 mm.

In some embodiments, the size of the gap D between the atomizing unit 101 and the liquid outlet is adjustable. The control unit of the atomizing device 100 may also be configured to adjust the size of the gap D between the atomizing unit 101 and the liquid outlet. In this way, the control unit can adapt to droplets of different sizes by adjusting the size of the gap D between the atomizing unit 101 and the liquid outlet 121. For example, when it is assumed that the gap D is 2 mm, a droplet of 20 microliters is formed each time and conveyed to the atomizing unit 101, and if the output flow rate of the liquid conveying unit 102 is unchanged and the gap D is adjusted to 1.5 mm, the relatively smaller volume of the droplet formed at the liquid outlet can be in contact with the atomizing unit 101 and transferred to the atomizing unit 101 at one time under the action of the atomizing unit 101 (e.g., surface tension or vibration), so as to be atomized by the atomizing unit 101. It can be understood that the relationship between the size of the gap D and the size of the conveyed liquid may be predetermined, for example, by experiments or theoretical calculations. In this way, according to a predetermined relationship between the size of the gap D and the size of the liquid, the amount of the one-time transfer to the atomizing unit 101 can be effectively controlled, so that the liquid storage amount of the atomizing unit 101 in the working state can be adjusted, and the atomization effect and the working efficiency are improved.

Still referring to FIG. 1, the liquid conveying unit 102 further includes a plunger 123 and a plunger rod 124 connected to the plunger 123, wherein the plunger 123 is slidably engaged with the inner wall of the liquid storage container 122, and the plunger 123 can be pushed to move in the liquid storage container 122 through the movement of the plunger rod 124, so as to output the liquid to be atomized from the liquid outlet 121. Compared with the liquid delivery system of the traditional atomizing device, the plunger-rod structure of the liquid conveying unit 102 can facilitate accurate quantitative control of the output flow, thereby completing the control of the delivery amount and delivery speed of the trace of liquid to be atomized. It should be noted that the liquid conveying unit 102 may also adopt other liquid delivery modes, such as a peristaltic pump, a syringe pump, a microfluidic pneumatic micropump, etc.

As shown in FIG. 1, the atomizing device 100 further includes a driving unit 104, and the driving unit 104 drives the plunger rod 124 to move via the transmission member 103, so as to accurately control the liquid delivery rate and/or delivery amount of the liquid conveying unit 102. In the embodiment shown in FIG. 1, the transmission member 103 includes a lead screw 131 and a lead screw nut 132, wherein the lead screw 131 is connected to the driving unit 104 and rotates under the driving of the driving unit 104. The driving unit 104 may adopt a stepping motor, an ultrasonic motor, or other mechanisms capable of driving the lead screw 131 to rotate. Rotation of the lead screw 131 causes the lead screw nut 132 to move in the axial direction of the lead screw 131, and due to the connection relationship between the lead screw nut 132 and the plunger rod 124, the lead screw nut 132 can drive the plunger rod 124 to controllably push the plunger 123 to move, so that a certain amount of liquid to be atomized is outputted from the liquid outlet 121. As shown in FIG. 1, the lead screw nut 132 may be connected to the holding member 133, while the plunger rod 124 is removably secured to the holding member 133, such a configuration may facilitate overall replacement of the liquid conveying unit 102. For example, the liquid conveying unit 102 packaged with a dose of medicine or vaccine may be detachably fixed in the holding member 133 to complete a certain dose of liquid delivery. After all the liquid is completely outputted, the old liquid conveying unit may be directly replaced with a new liquid conveying unit, so that liquid pollution and residual liquid waste that may be generated in the liquid replenishment process may be avoided.

In some embodiments, the liquid conveying unit 102 may further include a liquid level sensor, configured to detect an amount of liquid to be atomized in the liquid storage container 122. Further, the control unit may be in communication with the liquid level sensor and configured to generate an acoustic or optical warning signal when the liquid level sensor indicates that the liquid in the liquid storage container 122 is lower than a specified liquid level. It should be noted that the liquid level sensor may adopt a pressure type liquid level sensor or may be any other type of structure or component capable of monitoring or indicating the liquid level in the liquid storage container 122. For example, in some embodiments, the liquid level sensor may be a member that monitors the amount of motion of the plunger 123, the plunger rod 124, or the transmission member 103, which can determine the liquid level in the liquid storage container 122 by monitoring the amount of motion of the plunger or the like.

It can be understood that based on the driving of the driving unit 104 and/or the monitoring of the liquid level sensor, the amount of the liquid conveyed to the atomizing unit 101 by the liquid conveying unit 102 can be accurately controlled, which ensures the accuracy of the amount of the formed mist. For example, assuming that a total of 1 ml of liquid needs to be delivered to the atomizing unit 101 within 10 seconds, the liquid conveying unit 102 may deliver the liquid to be atomized to the atomizing unit 101 at a rate of 100 microliters per second, and the liquid to be atomized may be continuously conveyed to the liquid storage area of the atomizing unit 101 at a liquid amount of less than 10 microliters, that is, 10 or more droplets per second (continuously transferred droplets are more similar to the state of a liquid flow) are transferred to the atomizing unit 101. Since the atomizing unit 101 is synchronously atomized the liquid stored therein, the liquid level height in the liquid storage area of the atomizing unit may maintain a desired lower height in 10 seconds, for example, less than 2 mm.

Although not shown in FIG. 1, the atomizing device 100 may further include a mist temporary storage container. The mist temporary storage container has a mist inlet and a mist suction port, wherein the mist inlet is configured to be detachably engaged with the atomizing unit 101 to receive and temporarily store the mist atomized by the atomizing unit 101. For example, the atomizing unit 101 may be having a misting port, and the mist inlet of the mist temporary container may have a shape matching the misting port, so that the mist inlet may receive the mist atomized by the atomizing unit 101 from the misting port. After an atomization time or a primary atomization operation, when a certain amount or concentration of mist is stored in the mist temporary storage container, a user can inhale the mist stored in the mist temporary storage container through the mist suction port. Compared with a traditional manner of continuous inhalation or rhythmic inhalation, the mist temporary storage container can achieve inhalation of a drug with a certain concentration at a time, which is not only suitable for the use of micro-atomization delivery, but also effectively reduces the operation difficulty of a user and improves the efficiency of drug delivery.

In order to visually indicate the concentration of the liquid to be atomized in the mist temporary storage container, in some embodiments, the atomizing device 100 may further include a concentration mark disposed in the mist temporary storage container. Accordingly, the mist temporary container may include an observation part that can observe the concentration mark from the outside. The parameters such as the shape, configuration, color, position, and/or brightness of the concentration mark may be set such that when the mist concentration in the mist temporary container is below a predetermined concentration value, the concentration mark can be visually observed at the observation part, and when the mist concentration in the mist temporary container is higher than the predetermined concentration value, the concentration mark cannot be visually observed at the observation part. In some embodiments, the concentration mark may be a mark disposed on an inner wall of the mist temporary storage container, for example, a pattern or a concave/convex structure having a certain gray scale or color, and the observation part is formed by a light-transmissive material. In some other embodiments, the concentration mark may be disposed at a general center position of the mist temporary storage container, and the mist temporary storage container may include a plurality of observation parts in different directions, so as to facilitate observation by a user. Due to the existence of the concentration mark, the user can judge whether the concentration or total amount of the to-be-atomized liquid received by the user meets the requirement or not, so that the situation that the medicine or the vaccine is invalid due to insufficient inhalation amount is avoided.

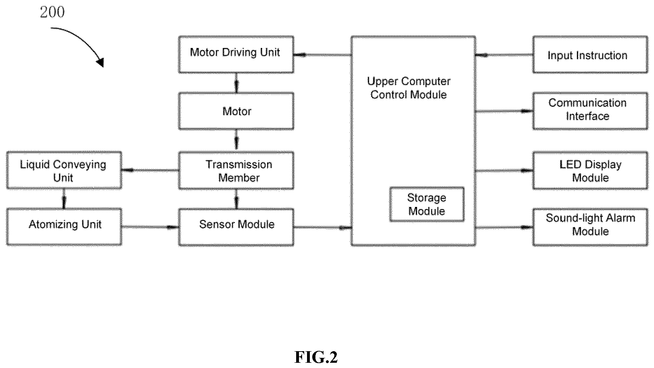

FIG. 2 illustrates a system block diagram of an atomizing device 200 according to another embodiment of the present disclosure. The system block diagram shown in FIG. 2 may correspond to the atomizing device 100 shown in FIG. 1. As shown in FIG. 2, the atomizing device 200 may include an upper computer control module, and the upper computer control module may include a storage module for storing instructions to be executed or related data. The upper computer control module may include a human-computer interaction interface, so that a user may input a specific working instruction. Meanwhile, the atomizing device 200 may further have an LED display module or a display panel to display various data information related to atomization, such as the liquid level in the liquid storage container, the usage time or the number of used times of the piezoelectric ceramic atomization piece, the operation data of the driving unit, and the like. In addition, the atomizing device 200 may further have a special sound-light alarm module, so that the atomizing device 200 can provide a warning of a corresponding sound signal or optical signal to an operator in time when the operation is abnormal (for example, the liquid level is low, the number of times the atomization piece is used, and the motor is abnormal). In some other embodiments, the upper computer control module may further communicate with an external device through a communication module (such as WiFi, ZigBee, RF, Bluetooth, etc.), so that remote control, as well as cloud analysis and processing of status data of the atomizing device 200 can be realized.

Still referring to FIG. 2, the atomizing device 200 sends an instruction to the motor driving module through the upper computer control module, so that the driving motor drives the motor to start, thereby driving the transmission member to move, and further driving the to-be-atomized liquid of the liquid conveying unit to be outputted to the atomizing unit for atomization. The atomizing device 200 may further include a sensor module, and the sensor module may sense data at the atomizing unit and the transmission member (for example, the amount of the liquid to be atomized driven by the transmission member, the operation state of the atomizing unit, the cumulative number of atomization times, the current liquid level of the liquid conveying unit, etc.), and transmit same to the upper computer control module. The upper computer control module may perform corresponding control operations according to the obtained information (for example, replacing the liquid conveying unit, replacing the atomizing unit, adjusting the liquid delivery flow rate, etc.). It should be noted that each unit or component in the atomizing device 200 may adopt the same structure as the atomizing device 100 shown in FIG. 1 or may adopt different structures.

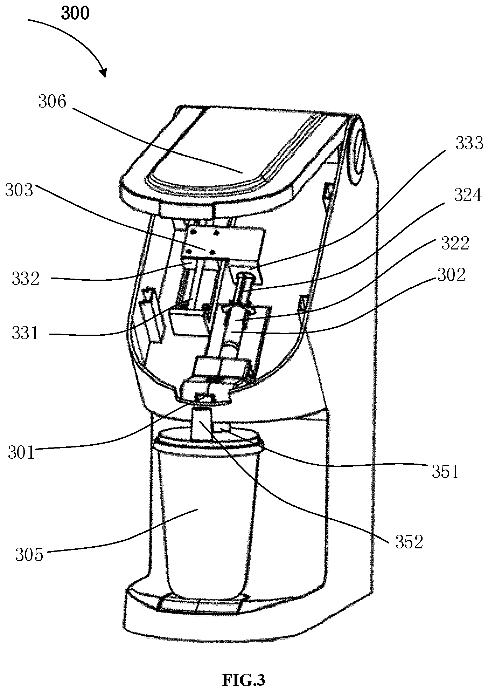

FIG. 3 shows a schematic diagram of an atomizing device 300 according to yet another embodiment of the present disclosure, the structure of which is similar to the structure of the atomizing device 100 shown in FIG. 1, and their members or components with similar symbols have similar structures or functions.

As shown in FIG. 3, the atomizing device 300 is having an atomizing unit 301 and a liquid conveying unit 302 for conveying liquid to be atomized to the atomizing unit 301, and the liquid atomizing unit 302 comprises a liquid storage container 322 and a plunger rod 324 for pushing the plunger in the liquid storage container to move so as to convey the liquid. When in operation, the driving unit (not shown in the figure) of the atomizing device 300 drives the plunger rod 324 to move via the transmission member 303 to realize liquid conveying. The transmission member 303 includes a lead screw 331 and a lead screw nut 332 matching the lead screw. One end of a holding member 333 is attached to the lead screw nut 332, and the other end is fixing to the plunger rod 324, so as to transmit the driving force from the lead screw nut 332 to the plunger rod 324. The structure of the transmission portion of the atomizing device 300 is generally similar to the transmission member 103 shown in FIG. 1, and details are not described herein again.

Still referring to FIG. 3, the atomizing device 300 further includes a mist temporary storage container 305, which includes a mist inlet 351 and a mist suction port 352. The mist inlet 351 may have a shape matching a misting port, or alternatively, an airtight structure (not shown) may be provided to connect the mist inlet 351 and the misting port. When in use, a user can place the mist temporary storage container 305 below the atomizing unit 301 to receive atomized mist, and when the mist concentration reaches a certain degree, the mist temporary storage container 305 is taken out, and the mist stored in the mist temporary storage container 305 is sucked through the mist suction port 352. The use of the mist temporary storage container 305 not only can ensure one-time high-concentration inhalation of the mist, but also avoids pollution and cross infection possibly caused in the use process of different users. As shown in FIG. 3, the atomizing device 300 may further include a cover plate structure 306 to effectively protect the atomizing unit, the liquid conveying unit, the driving unit, and the like. It may be understood that, in some embodiments, the mist inlet 351 and the mist suction port 352 may be the same opening.

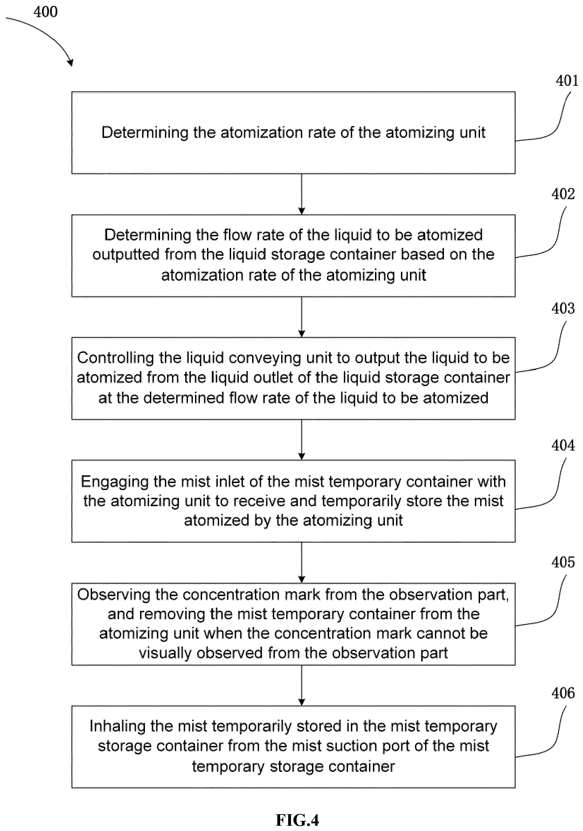

FIG. 4 illustrates a flowchart of a method 400 of using an atomizing device according to an embodiment of the present application. For ease of understanding, the usage method 400 will be specifically described below with reference to the atomizing device 300, and it can be understood that the usage method 400 may also be implemented by using an atomizing device with different structures or components comparing to the atomizing device 300. As shown in FIG. 4, in step 401, an atomization rate of the used atomizing unit 301 is determined. As described above, the atomization rate of the atomizing unit may be obtained in various manners, and details are not described herein again. It is assumed that the atomization rate of the determined atomizing unit 301 is 0.1 ml/min, and then in step 402, based on the determined 0.1 ml/min atomization rate of the atomizing unit 301, the flow rate of the to-be-atomized liquid outputted from the liquid storage container 322 can be correspondingly determined. Thereafter, in step 403, based on the determined flow rate of the liquid to be atomized, the liquid conveying unit 302 is controlled to output the liquid to be atomized at a corresponding flow rate. It should be noted that in some embodiments, the liquid conveying unit 302 may be controlled to output 0.01 ml to 2 ml of the liquid to be atomized within one minute, preferably 0.01 ml to 0.6 ml; in other embodiments, the liquid conveying unit 102 may also intermittently output 0.01 ml to 2 ml of the liquid to be atomized once, and the period of each liquid output is 1 to 30 seconds, preferably 2 to 10 seconds.

Although FIG. 3 is not shown, there may be a gap D between the atomizing unit 301 and the liquid outlet, the gap D enables the output liquid to be atomized to form a droplet of a predetermined size at the liquid outlet, and the droplet may be transferred to the atomizing unit 301 due to surface tension after being in contact with the atomizing unit 301. Such a gap size can avoid free falling due to gravity.

Still referring to FIGS. 3 and 4, in some embodiments, in step 404, the mist inlet 351 of the mist temporary container 305 is engaged with the atomizing unit 301 to receive and temporarily store the mist atomized by the atomizing unit 301. Subsequently, in step 405, the concentration mark (not shown in the figure) of the mist temporary storage container 305 is visually observed from the observation part, and when the concentration mark cannot be visually observed from the observation part, it is indicated that the concentration of the to-be-atomized liquid or the drug in the mist temporary storage container 305 reaches an inhalable requirement. Thereafter, in step 406, a certain concentration of the atomized liquid in the mist temporary storage container may be sucked in at one time from the mist suction port 352 of the mist temporary storage container 305, so as to achieve the best inhalation delivery effect.

### Example 1

In some embodiments, the liquid conveying unit of the atomizing device may be configured to intermittently convey the liquid to be atomized to the atomizing unit, and the atomizing unit is also configured to perform single atomization on the liquid to be atomized received each time. This type of single shot atomizing device is particularly suitable for fractionated micro-atomization delivery requirements, such as fractionated delivery of drugs or vaccine solutions. The following is the amount of atomized liquid medicine actually measured each time when a single shot atomizing device of this type of the present application is used to perform atomization processing with a target single atomization liquid medicine amount of 50 mg. The experiment used a single weighing method to obtain the actual amount of atomized liquid medicine each time. A total of three rounds of ten atomization measurements are performed, and the specific measurement data are respectively shown in Tables 1 to 3 as follows: it can be seen that the standard deviation of the amount of atomized liquid medicine is very small:

**TABLE 1. First round of measurement data**

| No. | Number of liquid delivery/atomization | Actual atomized liquid medicine |
|---|---|---|
| 1 | 1 | 53.9 |
| 2 | 1 | 53.7 |
| 3 | 1 | 52.6 |
| 4 | 1 | 54.4 |
| 5 | 1 | 51.3 |
| 6 | 1 | 54.4 |
| 7 | 1 | 52.3 |
| 8 | 1 | 53.6 |
| 9 | 1 | 53.0 |
| 10 | 1 | 51.9 |
| Average atomized liquid medicine | | 53.1 |
| Standard deviation | | 1.1 |
| Coefficient of variation | | 2.01% |

**TABLE 2. Second round of measurement data**

| No. | Number of liquid delivery/atomization | Actual atomized liquid medicine |
|---|---|---|
| 1 | 1 | 52.9 |
| 2 | 1 | 51.4 |
| 3 | 1 | 54.0 |
| 4 | 1 | 50.9 |
| 5 | 1 | 54.1 |
| 6 | 1 | 50.7 |
| 7 | 1 | 53.1 |
| 8 | 1 | 52.6 |
| 9 | 1 | 51.9 |
| 10 | 1 | 52.8 |
| Average atomized liquid medicine | | 52.4 |
| Standard deviation | | 1.2 |
| Coefficient of variation | | 2.27% |

**TABLE 3. Third round of measurement data**

| No. | Number of liquid delivery/atomization | Actual atomized liquid medicine |
|---|---|---|
| 1 | 1 | 53.3 |
| 2 | 1 | 50.6 |
| 3 | 1 | 53.8 |
| 4 | 1 | 52.2 |
| 5 | 1 | 52.6 |
| 6 | 1 | 52.7 |
| 7 | 1 | 51.7 |
| 8 | 1 | 53.0 |
| 9 | 1 | 51.6 |
| 10 | 1 | 52.2 |
| Average atomized liquid medicine | | 52.4 |
| Standard deviation | | 0.9 |
| Coefficient of variation | | 1.77% |

### Example 2

The following is the amount of atomized liquid medicine actually measured each time when a single shot atomizing device of this type of the present application is used to perform atomization processing with a target single atomization liquid medicine amount of 100 mg. The experiment also adopts a single weighing method to measure the amount of the actual atomized liquid medicine each time, and a total of three rounds of ten times of atomization measurement are performed. The specific measurement data are respectively shown in Tables 4 to 6 as follows, and it can be seen that the standard deviation of the amount of atomized liquid medicine is very small:

**TABLE 4. First round of measurement data**

| No. | Number of liquid delivery/atomization | Actual atomized liquid medicine |
|---|---|---|
| 1 | 1 | 106.5 |
| 2 | 1 | 108.2 |
| 3 | 1 | 105.4 |
| 4 | 1 | 105.0 |
| 5 | 1 | 104.0 |
| 6 | 1 | 105.0 |
| 7 | 1 | 105.1 |
| 8 | 1 | 105.5 |
| 9 | 1 | 102.1 |
| 10 | 1 | 101.0 |
| Average atomized liquid medicine | | 104.8 |
| Standard deviation | | 2.1 |
| Coefficient of variation | | 1.96% |

**TABLE 5. Second round of measurement data**

| No. | Number of liquid delivery/atomization | Actual atomized liquid medicine |
|---|---|---|
| 1 | 1 | 100.5 |
| 2 | 1 | 105.6 |
| 3 | 1 | 103.1 |
| 4 | 1 | 107.5 |
| 5 | 1 | 104.7 |
| 6 | 1 | 106.1 |
| 7 | 1 | 103.8 |
| 8 | 1 | 102.0 |
| 9 | 1 | 105.3 |
| 10 | 1 | 104.8 |
| Average atomized liquid medicine | | 104.3 |
| Standard deviation | | 2.1 |
| Coefficient of variation | | 1.97% |

**TABLE 6. Third round of measurement data**

| No. | Number of liquid delivery/atomization | Actual atomized liquid medicine |
|---|---|---|
| 1 | 1 | 104.7 |
| 2 | 1 | 104.1 |
| 3 | 1 | 103.7 |
| 4 | 1 | 100.0 |
| 5 | 1 | 102.8 |
| 6 | 1 | 102.3 |
| 7 | 1 | 103.7 |
| 8 | 1 | 102.1 |
| 9 | 1 | 104.3 |
| 10 | 1 | 105.5 |
| Average atomized liquid medicine | 103.3 | |
| Standard deviation | 1.6 | |
| Coefficient of variation | 1.52% | |

The atomizing device according to an embodiment of the present application is particularly suitable for delivering a trace of pulmonary inhalation liquid preparation, such as a drug or a vaccine. The atomizing device has the advantages of less residual liquid, no need of flushing, accurate quantification, wide application range, no microbial pollution, no blockage and the like. The atomization median particle size can reach 5 microns or below. In addition, since the atomizing device may further include a mist temporary storage container, a user can accurately and efficiently achieve suction of high-concentration drugs or vaccine mist, thereby achieving maximum utilization of drugs or vaccines.

The foregoing is an overview of the present application, which may be simplified, summarized, and omitted, and thus those skilled in the art will recognize that this portion is illustrative only and is not intended to limit the scope of the present application in any manner. This Summary is not intended to identify key features or essential features of the claimed subject matter, nor is it intended to be used as an auxiliary means to determine the scope of the claimed subject matter.

It should be noted that although several components or sub-components of the atomizing device are mentioned in the above detailed description, such partitioning is merely exemplary rather than mandatory. Indeed, according to an embodiment of the present application, the features and functions of the two or more components described above may be embodied in one component. Conversely, the features and functions of one of the components described above may be further divided into embodied by multiple components.

Those of ordinary skill in the art may understand and implement other changes to the disclosed embodiments by studying the specification, the disclosure, the drawings, and the appended claims. In the claims, the word "comprising" does not exclude other elements and steps, and the terms "a" and "an" do not exclude a plurality. In the practical application of the present application, a part may perform the functions of a plurality of technical features referred to in the claims. Any reference signs in the claims should not be construed as limiting the scope.

## Claims

1. An atomizing device, comprising:
a liquid conveying unit, wherein the liquid conveying unit comprises a liquid storage container having a liquid outlet, and the liquid conveying unit is configured to output a liquid to be atomized from the liquid outlet of the liquid storage container at a flow rate;
an atomizing unit, wherein the automizing unit is configured to receive the liquid to be atomized from the liquid outlet of the liquid storage container and atomize the liquid to be atomized into mist, wherein the atomizing unit has a predetermined atomization rate; and
a control unit, wherein the control unit is in communication with the liquid conveying unit, and the control unit is configured to control a flow rate at which the liquid conveying unit outputs the liquid to be atomized according to the predetermined atomization rate of the atomizing unit.

2. The atomizing device of claim 1, wherein a gap is formed between the liquid outlet of the liquid storage container and the atomizing unit, the gap allows the liquid to be atomized to form a droplet of a predetermined size at the liquid outlet, and the liquid droplet contacts the atomizing unit and is transferred to the atomizing unit.

3. The atomizing device of claim 2, wherein the gap between the atomizing unit and the liquid outlet is insufficient to enable the liquid to be atomized to form a droplet falling due to gravity.

4. The atomizing device of claim 2, wherein the control unit is configured to adjust a size of the gap between the atomizing unit and the liquid outlet.

5. The atomizing device of claim 4, wherein the control unit is configured to change the size of the droplet that can be formed at the liquid outlet by adjusting the size of the gap between the atomizing unit and the liquid outlet.

6. The atomizing device of claim 1, wherein the atomizing unit comprises a piezoelectric ceramic microporous atomization piece, the piezoelectric ceramic microporous atomization piece has a liquid storage area for receiving the liquid to be atomized from the liquid outlet of the liquid storage container, and the flow rate of the liquid to be atomized outputted by the liquid conveying unit is set such that a maximum height of a liquid level in the liquid storage area is 1 mm to 4 mm when the piezoelectric ceramic microporous atomization piece is in a continuous working state.

7. The atomizing device of claim 1, wherein the liquid conveying unit comprises a plunger and a plunger rod, wherein the plunger is slidably engaged with an inner wall of the liquid storage container, and the plunger rod is connected to the plunger and is configured to drive the plunger to move in the liquid storage container, so that the liquid to be atomized flows out of the liquid outlet.

8. The atomizing device of claim 7, wherein the device further comprises:
a driving unit, wherein the driving unit is configured to drive the plunger rod by a transmission member, wherein the transmission member comprises a lead screw and a lead screw nut, the driving unit is connected to the lead screw to drive the lead screw to rotate, and the plunger rod is connected to the lead screw nut and moves in an axial direction of the lead screw along with the lead screw nut.

9. The atomizing device of claim 1, wherein the device further comprises a mist temporary storage container having a mist inlet and a mist suction port, and the mist inlet is configured to be detachably engaged with the atomizing unit to receive and temporarily store the mist atomized by the atomizing unit.

10. The atomizing device of claim 9, wherein the device comprises a concentration mark arranged in the mist temporary storage container, and the mist temporary storage container comprises an observation part which can be observed from the outside of the mist temporary storage container; and wherein the concentration mark is set to be visually observed at the observation part when the mist concentration in the mist temporary storage container is lower than a preset concentration value, and not to be visually observed at the observation part when the mist concentration in the mist temporary storage container is higher than the preset concentration value.

11. The atomizing device of claim 10, wherein the concentration mark is a mark arranged on the inner wall of the mist temporary storage container, and the observation part is formed of a light-transmissive material.

12. The atomizing device of claim 1, wherein the liquid storage container of the liquid conveying unit comprises a liquid level sensor, the control unit is in communication with the liquid level sensor and is configured to generate a warning signal when the liquid level sensor indicates that the liquid in the liquid storage container is lower than a specified liquid level.

13. A method of using the atomizing device of any one of claims 1 to 12, the method comprising performing the following steps by the control unit:
determining an atomization rate of the atomizing unit;
determining the flow rate of the liquid to be atomized from the liquid outlet of the liquid storage container based on the atomization rate of the atomizing unit; and
controlling the liquid conveying unit to output the liquid to be atomized from the liquid outlet of the liquid storage container based on the determined flow rate of the liquid to be atomized.

14. The method of claim 13, wherein a gap is formed between the liquid outlet of the liquid storage container and the atomizing unit, the gap allows the liquid to be atomized to form a droplet of a predetermined size at the liquid outlet, and the droplet contacts the atomizing unit and is transferred to the atomizing unit, and the method comprises the following steps performed by the control unit:
adjusting a size of the gap between the atomizing unit and the liquid outlet to change the size of the droplet that can be formed at the liquid outlet.

15. The method of claim 13, wherein the atomizing device further comprises:
a mist temporary storage container having a mist inlet and a mist suction port, the mist inlet being configured to be detachably engaged with the atomizing unit to receive and temporarily store mist atomized by the atomizing unit; and
a concentration mark provided in the mist temporary storage container, and the mist temporary storage container comprises an observation part that can be observed from the outside of the mist temporary storage container; wherein the concentration mark is set to be visually observed at the observation part when the mist concentration in the mist temporary storage container is lower than a preset concentration value, and not to be visually observed at the observation part when the mist concentration in the mist temporary storage container is higher than the preset concentration value;
wherein the method further comprises:
engaging the mist inlet of the mist temporary storage container with the atomizing unit to receive and temporarily store mist atomized by the atomizing unit;
observing the concentration mark through the observation part, and removing the mist temporary storage container from the atomizing unit when the concentration mark cannot be visually observed from the observation part; and
sucking the mist temporarily stored in the mist temporary storage container from the mist suction port of the mist temporary storage container.
